Europäisches Patentamt

European Patent Office

Office européen des brevets

(11) Veröffentlichungsnummer : **0 071 152**
**B1**

(12) **EUROPÄISCHE PATENTSCHRIFT**

(45) Veröffentlichungstag der Patentschrift :
30.01.85

(21) Anmeldenummer : **82106518.2**

(22) Anmeldetag : **19.07.82**

(51) Int. Cl.⁴ : **G 01 N 33/62, G 01 N 27/48**

(54) Verfahren und Vorrichtung zur Bestimmung von Harnstoff.

(30) Priorität : 29.07.81 DE 3129988

(43) Veröffentlichungstag der Anmeldung :
09.02.83 Patentblatt 83/06

(45) Bekanntmachung des Hinweises auf die Patenterteilung : 30.01.85 Patentblatt 85/05

(84) Benannte Vertragsstaaten :
DE FR GB SE

(56) Entgegenhaltungen :
WO-A-81 /035 46
DE-A- 1 803 863
DE-B- 1 933 302
US-A- 3 922 205
US-A- 3 926 734

(73) Patentinhaber : Siemens Aktiengesellschaft
Berlin und München Wittelsbacherplatz 2
D-8000 München 2 (DE)

(72) Erfinder : Mund, Konrad, Dr.
Langenbrucker Weg 10
D-8521 Uttenreuth (DE)
Erfinder : Gebhardt, Ulrich
Zedernstrasse 18
D-8521 Langensendelbach (DE)
Erfinder : Luft, Günter, Dipl.-Ing.
Lindenstrasse 4
D-8560 Lauf (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft ein Verfahren zur Bestimmung der Harnstoffkonzentration in Flüssigkeiten sowie eine Vorrichtung zur Durchführung dieses Verfahrens, d. h., einen sogenannten Harnstoffsensor.

Die Bestimmung der Harnstoffkonzentration ist beispielsweise bei Nierenkranken von Bedeutung. Zur Entgiftung des Blutes dieser Personen, d. h. zum Entfernen des Harnstoffes aus dem Blut, sind derzeit drei Verfahren in Gebrauch : Hämodialyse, Hämofiltration und Peritonealdialyse. Dabei wird angestrebt, die bei diesen Behandlungsmethoden, d. h. bei der Blutreinigung, anfallenden harnstoffhaltigen Elektrolyte bzw. die Blutfiltrate vom Harnstoff zu befreien, um auf die bislang erforderlichen Dialyse- und Substitionslösungen verzichten zu können. Hierbei besteht dann aber Interesse daran, den Erfolg der Behandlung zu erkennen und deren Endpunkt anzuzeigen, was eine exakte Harnstoffbestimmung voraussetzt.

Neben den genannten, bereits eingeführten Verfahren werden auch neue Methoden zum Entfernen des Harnstoffes ausgearbeitet, beispielsweise die Elektroadsorption oder die sogenannte indirekte Harnstoffoxidation. Auch dabei ist es aber erforderlich, die Harnstoffkonzentration zu überwachen, was brauchbare Harnstoffsensoren erfordert.

Von Vorteil wäre es auch, wenn implantierbare Harnstoffsensoren zur Verfügung ständen, weil dann im Blut nierenkranker Patienten die Harnstoffkonzentration überwacht werden könnte. Beim Erreichen eines Grenzwertes könnte dabei ein Signal ausgelöst und dann eine Blutwäsche vorgenommen werden.

Auch für Patienten, die an Diabetes leiden, würde sich eine exakte Harnstoffbestimmung als vorteilhaft erweisen. Diesen Personen soll nämlich mit einer implantierbaren Insulin-Dosiereinrichtung geholfen werden, wobei ein Glucosesensor einen Regelkreis ermöglicht. Die Eichkurven von Glucosesensoren werden bislang jedoch noch durch Harnstoff beeinträchtigt und deshalb könnte durch eine Harnstoffbestimmung eine Verbesserung erreicht, d. h. der störende Einfluß korrigiert werden.

Bisher wird der Harnstoffgehalt in (Körper-) Flüssigkeiten meistens diskontinuierlich durch chemische Analysen bestimmt. Eine derartige Verfahrensweise ist jedoch unbefriedigend. Die Bestimmung der Harnstoff-konzentration kann zwar auch kontinuierlich mittels eines enzymatischen Sensors erfolgen, hierbei wird aber die Anwendung durch die Lebensdauer der Enzymmembran begrenzt und eine Implantation ist nicht möglich.

Aufgabe der Erfindung ist es, ein Verfahren zur Bestimmung der Harnstoffkonzentration in Flüssigkeiten anzugeben, das kontinuierlich arbeitet und empfindlich ist und auch über lange Zeit eine zuverlässige Ermittlung des Harnstoffgehaltes erlaubt.

Dies wird erfindungsgemäß dadurch erreicht, daß einer durch eine für Harnstoff durchlässige Membran von der harnstoffhaltigen Flüssigkeit getrennten Meßelektrode mittels Bezugs- und Gegenelektrode zyklisch potentiostatisch zwei Potentialwerte aufgeprägt werden, wobei das höhere Potential im Intervall $0,9\,V \leqslant \varphi/H_2\text{rev} \leqslant 2,0\,V$ liegt und das niedrigere Potential kleiner als $0,6\,V/H_2\text{rev}$ ist, und daß beim höheren Potential der innerhalb eines vorgegebenen Zeitintervalls fließende Strom als Meßsignal ausgewertet wird.

Das erfindungsgemäße Verfahren benutzt zur Harnstoff-bestimmung eine Elektrodenanordnung, wobei der Harnstoff an der Meßelektrode oxidiert wird ; es handelt sich hierbei also um ein elektrochemisches Verfahren. Die bei diesem Verfahren eingesetzte Membran dient zur Begrenzung der Diffusion. Die Membran soll dabei so feinporig sein, daß nur kleine Moleküle, wie Harnstoff, Glucose und Aminosäuren, in die Membranporen eindringen und zur Elektrode wandern, während verhindert wird, daß große Moleküle, wie Eiweißstoffe, zur Elektrodenoberfläche gelangen.

Beim erfindungsgemäßen Verfahren wird der innerhalb eines vorgegebenen Zeitintervalls, d. h. während der Meßperiode, fließende Strom als Meßsignal ausgewertet. Vorzugsweise wird dabei als Meßsignal die umgesetzte Ladung ermittelt, d. h. der während der Meßperiode fließende Strom wird integriert. Der ermittelten Ladung kann dann eine bestimmte Harnstoffkonzentration zugeordnet werden, da das Meßsignal proportional zur Harnstoffkonzentration ist.

Wesentlich beim erfindungsgemäßen Verfahren ist, daß der anodische Strom bei dem höheren Potential gemessen wird, bei welchem die Oxidation des Harnstoffes erfolgt. Dieses Potential liegt vorzugsweise etwa bei 1,6 V, gemessen gegen die reversible Wasserstoffelektrode ($H_2\text{rev}$). Anschließend wird der Meßelektrode dann ein niedrigeres Potential aufgeprägt, das vorzugsweise etwa bei $0,4\,V/H_2\text{rev}$ liegt. Hierbei werden die Elektrodenoberfläche und damit auch die Reaktionszentren von blockierenden Stoffen, die reduziert werden, befreit. Dabei wird das Potential der Meßelektrode vorteilhaft durch ein zyklisches Potentialsprungprogramm mit zwei Niveaus, dem Meß- und dem Regenerierungspotential, gesteuert. Die Dauer der Belastung ist abhängig vom Diffusionskoeffizienten und von der Dicke der Membran ; sie beträgt maximal 5 bis 10 Minuten.

Eine Vorrichtung zur Durchführung des erfindungsgemäßen Verfahrens, ein sogenannter (elektrochemischer) Harnstoffsensor, weist eine Meßelektrode, eine Gegenelektrode und eine Bezugselektrode sowie eine Membran auf, welche einen Diffusionskoeffizienten für Harnstoff $\leqslant 10^{-7}\,cm^2 \cdot s^{-1}$ besitzt ; die Membran ist dabei vor der Meßelektrode angeordnet. Mit einem derartigen Sensor ist eine selektive Harnstoffbestimmung möglich.

Vorzugsweise weist die Membran einen Diffusionskoeffizienten für Harnstoff von etwa $10^{-11}\,cm^2 \cdot s^{-1}$ auf. Es hat sich nämlich gezeigt, daß mit abnehmender Durchlässigkeit der Membran, d. h. geringer werdender Porengröße, sich das Verhältnis der Durchlässigkeiten von Harnstoff und Glucose, das im freien Elektrolyten 2 beträgt, ändert. Bei einem Diffusionskoeffizienten D für Glucose von ca. $10^{-8}\,cm^2 \cdot s^{-1}$ erreicht das D-Verhältnis (von Harnstoff zu Glucose) bereits den Wert 10. Bei $D = 10^{-10}\,cm^2 \cdot s^{-1}$ für Glucose ist das D-Verhältnis etwa 100. Bei derartigen Werten erfolgt mit Sicherheit keine Störung der Harnstoffbestimmung durch Glucose.

Die Dicke der Membran beträgt beim erfindungsgemäßen Harnstoffsensor vorzugsweise $\leqslant 10\,\mu m$. Auf diese Weise wird erreicht, daß die Ansprechzeit des Sensors klein ist. Von Vorteil ist es ferner, wenn der Widerstand der Membran nicht größer ist als $1\,k\Omega \cdot cm^2$, weil dann die Doppelschichtkapazität der Meßelektrode schnell umgeladen werden kann.

Vorteilhaft ist die Membran sowohl hydrophob als auch hydrophil, d. h. sie weist zum einen Teil einen hydrophoben und zum anderen Teil einen hydrophilen Charakter auf und ist somit quasi bivalent. Im allgemeinen besteht die Membran aus einem hydrophoben Polymeren, das partiell hydrophil ist. Bei derartigen Membranen werden die Diffusionskoeffizienten durch die Anzahl der hydrophilen Gruppen in den hydrophoben Polymerketten beeinflußt. Als hydrophile Gruppen kommen dabei Substituenten wie —OH, —COOH, —SO$_3$H und —NH$_3{}^+$ in Frage.

Die im erfindungsgemäßen Harnstoffsensor verwendeten Membranen weisen vorteilhaft einen Hydrophilierungsgrad H im Bereich von 1 : 15 und 1 : 100, vorzugsweise von 1 : 30 und 1 : 60, auf. Unter « Hydrophilierungsgrad » wird dabei das Verhältnis von hydrophilen Gruppen zu vorhandenen Kohlenstoffatomen verstanden. Bei Hydrophilierungsgraden im angegebenen Bereich wird die Diffusion von Harnstoff — verglichen mit derjenigen von Glucose und Aminosäuren — begünstigt.

Vorteilhaft bestehen die Membranen aus schwach hydrophiliertem Polysulfon, vorzugsweise aus schwach sulfoniertem Polysulfon. Derartige Polymere können durch Sulfonierung von Polysulfon mit Chlorsulfonsäure hergestellt werden.

Die Meßelektrode besteht beim erfindungsgemäßen Harnstoffsensor vorteilhaft aus Edelmetall, vorzugsweise aus Platin. Als Bezugselektrode wird im allgemeinen eine Silber/Silberchlorid-Elektrode verwendet. Die Gegenelektrode ist vorteilhaft eine Elektrode mit großer Doppelschichtkapazität, insbesondere eine aktivierte Glaskohlenstoffelektrode (vgl. dazu: DE-OS 26 13 072). Derartige Elektroden polarisieren nur wenig, unterbinden eine Gasentwicklung und unerwünschte elektrochemische Reaktionen und halten den Energiebedarf für den Sensor gering.

Außerdem kann hierbei die Funktion von Gegen- und Bezugselektrode vereinigt werden, d. h. die Gegenelektrode dient gleichzeitig als Bezugselektrode.

Anhand von Ausführungsbeispielen und Figuren soll die Erfindung noch näher erläutert werden.

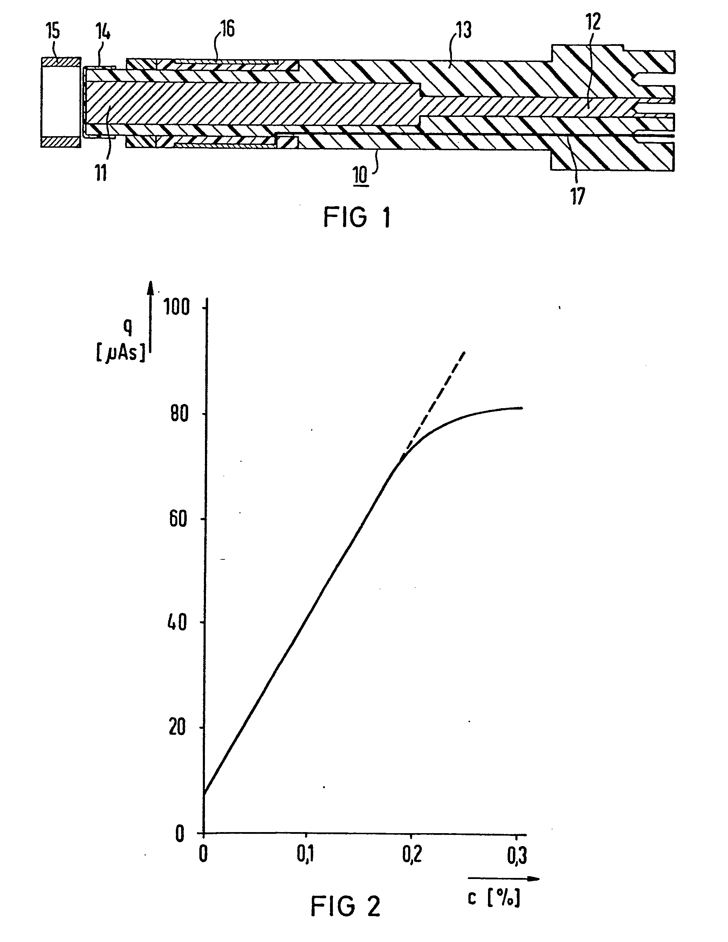

In Fig. 1 ist eine vorteilhafte Ausführungsform des erfindungsgemäßen Harnstoffsensors im Schnitt dargestellt. Der stabförmig ausgebildete Harnstoffsensor 10 weist eine zentral angeordnete Meßelektrode 11 in Form eines Platinstiftes auf. Die Meßelektrode 11, welche einen zur Kontaktierung dienenden Ansatz 12 aufweist, ist von einem isolierenden Kunststoffmantel 13, beispielsweise aus Polymethacrylat, umhüllt, wobei aber das vom Ansatz 12 abgewandte Ende des Platinstiftes frei liegt. An diesem Ende ist der Harnstoffsensor mit einer Membran 14 versehen, welche die freie Oberfläche der Meßelektrode 11 abdeckt. Zur festen Fixierung der Membran 14 dient, wie in Fig. 1 dargestellt ist, eine (ringförmige) Spannkappe 15. Außer der Meßelektrode 11 ist in die Kunststoffumhüllung 13 noch eine ringförmige Gegenelektrode 16 eingelagert, und zwar in der Weise, daß die äußere Oberfläche frei liegt, d. h. mit der Umgebung in Berührung steht. Die Gegenelektrode 16 besteht aus Glaskohlenstoff und fungiert gleichzeitig auch als Bezugselektrode. Zur Kontaktierung der Gegenelektrode 16 dient ein Draht 17.

Ein Sensor der vorstehend genannten Art wurde zur Bestimmung der Harnstoffkonzentration verwendet. Um die durchgeführten Versuche den physiologischen Bedingungen, insbesondere der Blutströmung, weitgehend anzupassen, wurde der Sensor in eine Durchflußapparatur eingebaut, wobei die Meßelektrode der Strömung ausgesetzt wurde, die Gegenelektrode somit strömungsmäßig hinter der Meßelektrode angeordnet war. Als Elektrolytflüssigkeit diente Tyrode-Lösung, eine mit Blut isotonische Lösung (enthaltend NaCl, KCl, CaCl$_2$, MgCl$_2$, NaH$_2$PO$_4$, NaHCO$_3$ und Glucose in destilliertem Wasser) ; diese Lösung wurde mit einem Gasgemisch, bestehend aus 95 % Preßluft und 5 % Kohlendioxid, gespült.

Bei den Messungen selbst wird die Meßelektrode für eine bestimmte Zeit, beispielsweise für 50 s, bei einem Potential von 1 600 mV/H$_2$rev belastet. Anschließend wird auf ein Potential von 400 mV/H$_2$rev gesprungen, das ebenfalls für 50 s an der Elektrode anliegt. Hierbei werden die an der Elektrode gebildeten Substanzen reduziert. Der Harnstoff, der bei diesem Potential nicht oxidiert wird, diffundiert an die freie Elektrodenoberfläche und wird dort adsorbiert. Beim Sprung auf das Potential von 1 600 mV/H$_2$rev (Belastungsdauer : 50 s) wird dann der Harnstoff — nach « Umladung » der Elektrodenkapazitäten — elektrochemisch oxidiert. Der dabei fließende Strom wird integriert, das erhaltene Integral dient als Meßwert. Der Meßwert wird dabei beispielsweise durch Integration der letzten 20 s erhalten.

In Fig. 2 ist eine aus Versuchen der vorstehend genannten Art unter Verwendung verschiedener Harnstoffkonzentrationen resultierende Eichkurve dargestellt ; dabei ist auf der Ordinate die Ladung q (in $\mu$As) aufgetragen und auf der Abszisse die Harnstoffkonzentration c (in %). Aus Fig. 2 ist ersichtlich, daß die ermittelte Ladung der Harnstoffkonzentration etwa bis zu einem Wert von 0,2 %, d. h. 200 mg/dl, proportional ist. Dieser Wert ist ungefähr das 10fache der mittleren physiologischen Konzentration von Harnstoff im menschlichen Körper.

Bei den vorstehend genannten Versuchen wurde als Meßelektrode eine platinierte Platinelektrode mit einer (freien) Fläche von 0,125 cm² eingesetzt. Als Membran diente eine sulfonierte Polysulfonmembran, welche einen Sulfonierungsgrad S von 0,175 aufwies. Unter « Sulfonierungsgrad » wird dabei im Rahmen der vorliegenden Patentanmeldung das Verhältnis von Sulfonsäuregruppen zu Sulfongruppen verstanden ; bei einem Verhältnis von 1 : 1 beträgt der Sulfonierungsgrad S = 1. Bei einer Membran mit S = 0,175 beträgt der Diffusionskoeffizient für Harnstoff ca. $3 \cdot 10^{-11}$ cm² $\cdot$ s⁻¹ und derjenige für Glucose ca. $4 \cdot 10^{-13}$ cm² $\cdot$ s⁻¹. Werden dichtere Membranen eingesetzt, d. h. Membranen mit einem geringeren Sulfonierungsgrad (und somit stärker ausgeprägtem hydrophoben Charakter), so ergibt sich auch noch bei höheren Harnstoffkonzentrationen eine Proportionalität zwischen Ladung und Konzentration.

Um festzustellen, inwieweit körpereigene Stoffe die Messungen der Harnstoffkonzentration beeinflussen, wurden dem Elektrolyten — neben Harnstoff — Glucose bzw. Aminosäuren zugesetzt. Dabei zeigte sich, daß Glucose (Zugabe ; 0,1 %) die Harnstoffbestimmung nicht beeinflußt. Die Zugabe von Aminosäuren mit der mittleren physiologischen Konzentration verursacht einen Meßfehler von ca. 5 %. Eine derartig geringfügige Abweichung ist aber tolerierbar. Es zeigt sich somit, daß mit dem erfindungsgemäßen Sensor eine selektive Bestimmung von Harnstoff in (Körper-)Flüssigkeiten möglich ist.


**Ansprüche**

1. Verfahren zur Bestimmung der Harnstoffkonzentration in Flüssigkeiten, dadurch gekennzeichnet, daß einer durch eine für Harnstoff durchlässige Membran von der harnstoffhaltigen Flüssigkeit getrennten Meßelektrode mittels Bezugs- und Gegenelektrode zyklisch potentiostatisch zwei Potentialwerte aufgeprägt werden, wobei das höhere Potential im Intervall $0,9 \, V \leq \varphi/H_2rev \leq 2,0 \, V$ liegt und das niedrigere Potential kleiner als 0,6 V/H₂rev ist, und daß beim höheren Potential der innerhalb eines vorgegebenen Zeitintervalls fließende Strom als Meßsignal ausgewertet wird.

2. Verfahren nach Anspruch 1, dadurch gekennzeichnet, daß als Meßsignal die umgesetzte Ladung ermittelt wird.

3. Verfahren nach Anspruch 1 oder 2, dadurch gekennzeichnet, daß das Potential der Meßelektrode durch ein zyklisches Potentialsprungprogramm mit zwei Niveaus gesteuert wird.

4. Verfahren nach einem der Ansprüche 1 bis 3, dadurch gekennzeichnet, daß das höhere Potential bei 1,6 V/H₂rev und das niedrigere Potential bei 0,4 V/H₂rev liegt.

5. Harnstoffsensor zur Durchführung des Verfahrens nach einem oder mehreren der Ansprüche 1 bis 4, gekennzeichnet durch eine Meßelektrode, eine Gegenelektrode und eine Bezugselektrode sowie durch eine von der Meßelektrode angeordnete Membran mit einem Diffusionskoeffizienten für Harnstoff $< 10^{-7}$ cm² $\cdot$ s⁻¹.

6. Harnstoffsensor nach Anspruch 5, dadurch gekennzeichnet, daß die Membran einen Diffusionskoeffizienten für Harnstoff von etwa $10^{-11}$ cm² $\cdot$ s⁻¹ aufweist.

7. Harnstoffsensor nach Anspruch 5 oder 6, dadurch gekennzeichnet, daß die Membran eine Dicke $\leq 10 \, \mu m$ aufweist.

8. Harnstoffsensor nach einem der Ansprüche 5 bis 7, dadurch gekennzeichnet, daß die Membran sowohl hydrophobe als auch hydrophile Eigenschaften besitzt.

9. Harnstoffsensor nach Anspruch 8, dadurch gekennzeichnet, daß der Hydrophilierungsgrad H der Membran im Bereich von $1 : 15 \geq H \geq 1 : 100$, vorzugsweise im Bereich von $1 : 30 \geq H \geq 1 : 60$, liegt.

10. Harnstoffsensor nach Anspruch 8 oder 9, dadurch gekennzeichnet, daß die Membran aus schwach hydrophiliertem Polysulfon, vorzugsweise sulfoniertem Polysulfon, besteht.

11. Harnstoffsensor nach einem oder mehreren der Ansprüche 5 bis 10, dadurch gekennzeichnet, daß die Meßelektrode aus Edelmetall, vorzugsweise aus Platin, besteht.

12. Harnstoffsensor nach einem oder mehreren der Ansprüche 5 bis 11, dadurch gekennzeichnet, daß die Gegenelektrode gleichzeitig als Bezugselektrode dient.

13. Harnstoffsensor nach Anspruch 12, dadurch gekennzeichnet, daß die Gegenelektrode aus Glaskohlenstoff besteht.


**Claims**

1. A method for determining the urea concentration in liquids, characterised in that, by means of a reference electrode and a counter-electode, two potential values are applied, cyclically and potentiostatically, to a measuring electrode which is separated from the urea-containing liquid by a membrane which is permeable to urea, the higher potential lying in the range $0.9 \, V \leq \varphi/H_2rev \leq 2.0 \, V$ and the lower potential being less than 0.6 V/H₂rev ; and that at the higher potential, the current which flows within a predetermined time period is evaluated as the measurement signal.

2. A method as claimed in Claim 1, characterised in that the converted charge is determined as the measurement signal.

3. A method as claimed in Claim 1 or Claim 2, characterised in that the potential of the measuring electrode is controlled by means of a cyclic potential jump programme having two levels.

4. A method as claimed in one of Claims 1 to 3, characterised in that the higher potential is 1.6 V/H$_2$rev and the lower potential is 0.4 V/H$_2$rev.

5. A urea sensor for carrying out the method claimed in one or more of Claims 1 to 4, characterised by a measuring electrode, a counter-electrode, and a reference electrode, and by a membrane which is arranged before the measuring electrode and which has a diffusion coefficient for urea of $< 10^{-7}$ cm$^2 \cdot$ s$^{-1}$.

6. A urea sensor as claimed in Claim 5, characterised in that the membrane has a diffusion coefficient for urea of about $10^{-11}$ cm$^2 \cdot$ s$^{-1}$.

7. A urea sensor as claimed in Claim 5 or Claim 6, characterised in that the membrane has a thickness of $\leq 10$ μm.

8. A urea sensor as claimed in one of Claims 5 to 7, characterised in that the membrane has both hydrophobic and hydrophilic properties.

9. A urea sensor as claimed in Claim 8, characterised in that the degree H to which the membrane is hydrophilic is in the range from $1 : 15 \geq H \geq 1 : 100$, preferably in the range from $1 : 30 \geq H \geq 1 : 60$.

10. A urea sensor as claimed in Claim 8 or Claim 9, characterised in that the membrane consists of polysulphone which is weakly hydrophilic, preferably sulphonated polysulphone.

11. A urea sensor as claimed in one or more of Claims 5 to 10, characterised in that the measuring electrode consists of a noble metal, preferably platinum.

12. A urea sensor as claimed in one or more of Claims 5 to 11, characterised in that the counter-electrode serves simultaneously as the reference electrode.

13. A urea sensor as claimed in Claim 12, characterised in that the counter-electrode consists of vitreous carbon.

**Revendications**

1. Procédé de détermination de la concentration d'urée dans des liquides, caractérisé en ce qu'il consiste à appliquer à une électrode de mesure séparée du liquide contenant de l'urée, par une membrane perméable à l'urée, cycliquement et d'une manière potentiostatique, deux valeurs de potentiel au moyen d'une électrode de référence et d'une contre-électrode, le potentiel le plus élevé étant compris entre $0,9$ V $\leq \Phi/H_2$rev $\leq 2,0$ V, et le potentiel le plus bas étant inférieur à 0,6 V/H$_2$rev, et à exploiter comme signal de mesure le courant électrique s'écoulant pendant un intervalle de temps prescrit au potentiel le plus élevé.

2. Procédé suivant la revendication 1, caractérisé en ce qu'il consiste à déterminer comme signal de mesure la charge transformée.

3. Procédé suivant la revendication 1 ou 2, caractérisé en ce qu'il consiste à commander le potentiel de l'électrode de mesure par un programme à saut de potentiel cyclique à deux niveaux.

4. Procédé suivant l'une des revendications 1 à 3, caractérisé en ce que le potentiel le plus élevé est à 1,6 V/H$_2$rev et le potentiel le plus bas est à 0,4 V/H$_2$rev.

5. Détecteur d'urée pour la mise en œuvre du procédé suivant l'une ou plusieurs des revendications 1 à 4, caractérisé par une électrode de mesure, une contre-électrode et une électrode de référence, ainsi que par une membrane disposée devant l'électrode de mesure et ayant un coefficient de diffusion pour l'urée inférieur à $10^{-7}$ cm$^2 \cdot$ s$^{-1}$.

6. Détecteur d'urée suivant la revendication 5, caractérisé en ce que la membrane a un coefficient de diffusion pour l'urée de $10^{-11}$ cm$^2 \cdot$ s$^{-1}$ environ.

7. Détecteur d'urée suivant la revendication 5 ou 6, caractérisé en ce que la membrane a une épaisseur inférieure ou égale à 10 microns.

8. Détecteur d'urée suivant l'une des revendications 5 à 7, caractérisé en ce que la membrane possède des propriétés aussi bien hydrophobes qu'hydrophiles.

9. Détecteur d'urée suivant la revendication 8, caractérisé en ce que le taux d'hydrophilisation H de la membrane est dans la plage de $1 : 15 \geq H \geq 1 : 100$, et de préférence dans la plage de $1 : 30 \geq H \geq 1 : 60$.

10. Détecteur d'urée suivant la revendication 8 ou 9, caractérisé en ce que la membrane est constituée d'une polysulfone, faiblement hydrophilisée, et de préférence d'une polysulfone sulfonée.

11. Détecteur d'urée suivant l'une ou plusieurs des revendications 5 à 10, caractérisé en ce que l'électrode de mesure est en un métal noble, de préférence en platine.

12. Détecteur d'urée suivant l'une ou plusieurs des revendications 5 à 11, caractérisé en ce que la contre-électrode sert en même temps d'électrode de référence.

13. Détecteur d'urée suivant la revendication 12, caractérisé en ce que la contre-électrode est en carbone vitreux.

FIG 1

FIG 2